# EUROPEAN PATENT APPLICATION

(11) **EP 2 399 591 A1**
(43) Date of publication of application: **28.12.2011**
(21) Application number: 10305684.2
(22) Date of filing: 25.06.2010
(51) Int. Cl.: A61K 31/727, A61P 7/02

(54) **Semuloparin for the extended prevention of a mortality and/or morbidity event in a patient having undergone hip fracture surgery**

(71) Applicant: Aventis Pharma S.A., 92160 Antony (FR)
(72) Inventor: The designation of the inventor has not yet been filed
(74) Representative: Romanowski, Caroline

(57) **Abstract**

The invention relates to the use of semuloparin or a pharmaceutically acceptable salt thereof for the extended prevention of a mortality and/or morbidity event, more specifically venous thromboembolism and death, in a patient having undergone hip fracture surgery.

## Description

The invention relates to the use of semuloparin or a pharmaceutically acceptable salt thereof for the extended prevention of a mortality and/or morbidity event, more specifically venous thromboembolism and death, in a patient having undergone hip fracture surgery.

Semuloparin, or AVE5026 (sanofi-aventis laboratory code) belongs to a new generation of hemisynthetic heparins. It is a new ultra-low molecular weight heparin, with an average molecular weight of 2000-3000 Daltons and a novel antithrombotic profile resulting from high anti-Factor Xa activity (- 160 U/mg) and residual anti-Factor IIa activity (- 2 U/mg), as described in Journal of Thrombosis and Haemostasis, 2009, vol. 7, 1143-1151. It is obtained by selective and controlled depolymerization of heparin, as described in the patent application WO 2004/033503 and in J. Thromb Haemost. (ibid). Semuloparin, in the form of its sodium salt, is in clinical development for venous thromboembolism prevention.

At the present time, low-molecular-weight heparins (LMWHs), the synthetic pentasaccharide fondaparinux or dose-adjusted anti-vitamin K are the primary treatments for the prevention of venous thromboembolic diseases.

Patients undergoing surgery are at substantial risk of postoperative venous thromboembolism (hereafter "VTE"). Many hospitalized patients also have additional risk factors for VTE. In addition, the risk of VTE persists several weeks after hospital discharge.

In order to avoid postoperative venous thromboembolic complication, guidelines in thrombosis (ACCP guidelines) recommend the use of antithrombotic drugs for certain categories of surgical patients. Amongst these drugs, the LMWH enoxaparin is the pharmacological VTE prevention agent with the highest clinical documentation in surgical populations and with the largest clinical use in this setting. Enoxaparin has an average molecular weight of 3800-5000 Daltons, an anti-Factor Xa activity comprised between 90 and 125 IU/mg and an anti-Factor IIa activity of 20-35 IU/mg.

The Applicant has now found that additional 3-week treatment, over the standard 7-10 day treatment, of a product outside of the LMWH class, namely the ultra-low molecular weight heparin (ULMWH) semuloparin, allows to reduce the occurrence of mortality and/or morbidity events in patients having undergone hip fracture surgery.

Therefore, the subject-matter of the invention is semuloparin or a pharmaceutically acceptable salt thereof, for use in the extended prevention of a mortality and/or morbidity event in a patient having undergone hip fracture surgery, wherein said event is selected from venous thromboembolism and death and wherein the efficacy of said use is clinically proven by a phase III clinical trial.

According to the present invention, the terms below have the following meanings:
- "extended prevention" refers to the prevention of venous thromboembolic events in a timeframe of several weeks, more specifically 4 weeks, after the hip fracture surgery ;
- "a patient" refers to a patient being at risk of VTE and for whom prophylaxis of venous thromboembolic events is indicated;
- "death" refer to all causes of death;
- "phase III clinical trial" refers to a multicenter, randomized, double-blinded study involving a large patients group (at least several hundreds of patients), aiming at being the definitive assessment of the effectiveness of the drug.

As used herein, the wording "semuloparin for use in the extended prevention of ..." shall be understood as being equivalent to the wording "use of semuloparin for the extended prevention of ..." or "use of semuloparin for the preparation of a medicament for use in the extended prevention of ...".

According to the instant invention, semuloparin designates the sodium salt of this ULMWH or any other pharmaceutically acceptable salt thereof.

More specifically, the subject-matter of the invention is semuloparin for use in the extended prevention of a mortality and/or morbidity event in a patient having undergone hip fracture surgery, being at risk of VTE and for whom prophylaxis of venous thromboembolic events is indicated, wherein said mortality and/or morbidity event is selected from VTE and death and wherein the efficacy of said use is clinically proven by a phase III clinical trial.

In particular, the subject-matter of the invention is semuloparin for its use as described above, wherein said mortality and/or morbidity event is selected from deep vein thrombosis (hereafter "DVT", including proximal or distal DVT), non-fatal pulmonary embolism and death.

In the framework of the present invention, the terms below have the following meanings:
- "deep vein thrombosis" : designates a blood clot in a deep vein of the lower limbs;
- "proximal DVT" : designates a DVT event occurring above the knee;
- "distal DVT" : designates a DVT event occurring below the knee.

According to the invention, semuloparin is administered for 26 to 33 days to the patient having undergone surgery (starting from the day of surgery).

Indeed, it has been found that administration of semuloparin for about 3-weeks (19-23 days), to patients who have already received approximately 1 week (7-10 days) treatment with semuloparin for VTE prevention after hip fracture surgery, enables to decrease the occurrence of VTE and death in these patients, compared to the occurrence of such events in patients who received the initial 1 week treatment but not the 3-week extension. "Treatment" is understood herein as a venous thromboprophylactic treatment.

In another embodiment, the invention relates to a method for the prevention of a mortality and/or morbidity event in a patient having undergone hip fracture surgery, which comprises the administration of an effective dose of semuloparin or a pharmaceutically acceptable salt thereof to a patient in need thereof, wherein said event is selected from venous thromboembolism and death and wherein the efficacy of said use is clinically proven by a phase III clinical trial, as described above.

According to the instant invention, semuloparin is administered at a 20 mg daily dose to patients with normal renal function or to patients with mild or moderate renal impairment. For patients with severe renal impairment, semuloparin is administered at a 10 mg daily dose.

According to the instant invention, the renal function of the patients is defined according to estimated creatinine clearance (CLcr) values calculated using the well-known Cockroft-Gault formula, and is classified according to the following characteristics :
- normal renal function: CLcr > 80 mL/min;
- mild renal impairment: 50 ≤ CLcr ≤ 80 mL/min;
- moderate renal impairment: 30 ≤ CLcr < 50 mL/min;
- severe renal impairment: CLcr < 30 mL/min.

The treatment with semuloparin is advantageously administered once daily. As used therein, "daily" means an administration every 24 hours plus or minus 4 hours.

Said treatment is advantageously administered for 26 to 33 days, as described above (7-10 days of initial treatment, followed by 19-23 days of extended prophylaxis).

The prevention of the mortality and/or morbidity event according to the instant invention is assessed at the end of the 19-23 days period of extended prophylaxis, i.e. from 26 to 33 days after start of the treatment with semuloparin (which corresponds to the day of surgery).

The invention also relates to the use of semuloparin or a pharmaceutically acceptable salt thereof for the manufacture of a medicament useful for the extended prevention of a mortality and/or morbidity event in a patient having undergone hip fracture surgery, wherein said event is selected from venous thromboembolism and death and wherein the efficacy of said use is clinically proven by a phase III clinical trial.

Having now described the present invention, the same will be more clearly understood by reference to the following examples of the invention, which are included herewith for purposes of illustration only and are not intended to be limiting of the invention.

The following abbreviations shall be used:
CLcr: Creatinine Clearance
DVT: deep vein thrombosis
IP: investigational product
PE: pulmonary embolism
UFH: unfractionated heparin
LMWH: low molecular weight heparin
OR: Odds Ratio
q.d.: *quaque die* (once daily)
s.c.: subcutaneously
SRI: severe renal impairment
VTE: venous thromboembolism
95% exact Cl: 95% exact Confidence Interval
95% mid-p Cl: 95% mid-p Confidence Interval

### Example 1): Preparation of semuloparin

Semuloparin, in the form of a sodium salt, is obtained by a chemoselective depolymerization of heparin, activated through its benzyl ester derivative, by the phosphazene base 2-tert-butylimino-2-diethylamino-1,3-dimethylperhydro-1,2,3-diazaphosphorine (BEMP). The hemisynthetic pathway, allowing to recover semuloparin in the form of a sodium salt, is described in the patent application WO 2004/033503 and in Journal of Thrombosis and Haemostasis, 2009, vol. 7, 1143-1151. This procedure yields semuloparin with an average molecular weight around 2400 Da, an anti-Factor Xa activity of - 160 U/mg and a residual anti-Factor IIa activity (∼2 U/mg).

**Example 2): The SAVE-HIP3 study.** A Multinational, Multicenter, Randomized, Double Blind Study comparing the Efficacy and Safety of AVE5026 with placebo for the Extended Prevention of Venous Thromboembolism in Patients Having Undergone Hip Fracture Surgery.

### 1) Study objectives

The primary objective of the study is to compare the efficacy of once daily (q.d.) subcutaneous (s.c.) injections of 20 mg AVE5026 (10 mg for patients with SRI) with placebo q.d. s.c. during 3 additional weeks following initial venous thromboprophylaxis for 7 to 10 days with open-label q.d. s.c. injections of 20 mg of AVE5026 for the prevention of venous thromboembolic events in patients having undergone hip fracture surgery. The secondary objective of this study is to evaluate the safety of extended AVE5026 prevention in patients having undergone hip fracture surgery.

### 2) Study design

This is a multicenter, multinational, randomized, double-blind, superiority study conducted in two parallel groups (AVE5026 and placebo) after an initial open-label phase with AVE5026 for 7 to 10 days in patients who have undergone a standard surgery for fracture of the upper third of the femur, including femoral head and neck.

During a screening period (from hospital admission to 8 ± 1 hours after incision closure), all patients hospitalized for hip fracture surgery are screened for participation. If they meet primary eligibility criteria (selection criteria for enrolment and exclusion criteria described below), they are asked to participate.

A patient is eligible for a post-operative enrolment in the first period of the trial (run-in phase) if the post-operative hemostasis has been established just prior to the first injection of AVE5026. The first injection of AVE5026 in the run-in phase is administered 8 ± 1 hours after incision closure then patients are treated with once daily subcutaneous injection (pre-filled syringes) of AVE5026 up to Day 7-10 of the run-in phase (AVE5026 20 mg if their CLcr is ≥ 30 mL/min and 10 mg if their CLcr < 30mL/min).

At Day 7-10 of the run-in phase (Run-in D7-10), the Investigator checks that all eligibility criteria for randomization described below are met. If they are satisfied, the patient is randomized for participating in the double-blind treatment phase.

Randomized treatment is allocated to eligible patients through a centralized randomization system. The randomization is stratified by geographical region and by the estimated CLcr at randomization (< 30 or ≥ 30 mL/min).

IP injections (AVE5026 or placebo) are done once daily subcutaneously (pre-filled syringes) from Day 1 up to Day 19-23 of the double-blind treatment phase.

During the double-blind treatment phase of the study, if an Investigator requires diagnostic test(s) for VTE, these tests are performed and included in an adjudication dossier that is to be submitted to a blinded Central Independent Adjudication Committee (CIAC) that shall confirm or rule out the presence of VTE.

A mandatory bilateral venography of the lower limbs is performed at Day 19-24 of the double-blind treatment phase. The results of venography are adjudicated by the CIAC.

All bleeding events and deaths reported from randomization up to the end of the study are adjudicated.

During the run-in phase, adverse events (including VTE, bleedings), concomitant medications and compliance are assessed.

During the double-blind treatment phase, VTE, bleedings and adverse events, concomitant medications and compliance are assessed.

An end of treatment visit is performed on the day of last IP injection or at Day 23 of the double-blind treatment phase, whichever comes first.

A follow-up visit is scheduled at Day 49-53 post-randomization (Day 1 is defined as Day 1 of the double-blind treatment phase). During this visit, information regarding adverse events (including bleedings and VTE) is collected.

Maximum duration of study participation is 63 days, including a run-in phase (7-10 days), a double-blind treatment phase (19-23 additional days from randomization day) and a follow-up period with a visit at Day 49-53 after randomization (Day 1 being the day of randomization). The study is considered completed for a patient at the time he/she completes the follow-up visit 28±3 days after study drug discontinuation.

### 3) Patients

A total of 469 patients are randomized in the study, at the end of the run-in phase.

Patients meeting the following inclusion criteria are suitable for enrolment in the run-in phase:
- Standard surgery for fracture of the upper third of the femur, including femoral head and neck with incision closure performed 8 ± 1 hours previously, and provided that hemostasis has been established.
- Signed written informed consent.

Patients are randomized at the end of the run-in phase only if they satisfy the following inclusion criteria for randomization:
- Patients who completed the run-in phase without permanent discontinuation of AVE5026 prevention,
- and still complying with the selection criteria of the run-in phase.

The exclusion criteria are checked before the enrolment in the run-in phase and before randomization into the double-blind treatment phase. Patients meeting one of the following criteria are excluded from enrolment into the study:
1. Legal lower age limitations (country specific).
2. Estimated time of injury/fracture before hospital admission > 24 hours.
3. Time from hospital admission to surgery > 36 hours (if time from injury to hospital admission is documented to be less than 24 hours, this 36 hours time window can be expanded but in all cases the time from injury to surgery must not exceed 60 hours).
3A1. Multiple trauma affecting more than one organ system.
4. Any major orthopedic surgery within 3 months prior to enrollment.
5. Hemorrhagic stroke or recent (less than 3 months prior to enrollment) brain, spinal or ophthalmological surgery.
6. Active or recent (<3 months) significant bleeding, including gastro-intestinal bleeding.
7. Hemostasis prior to the first AVE5026 injection of the run-in phase not established.
8. Clinical signs or symptoms of DVT or PE within the last 12 months or known post-phlebitic syndrome.
9. Known sensitivity to iodine or contrast dyes, and any contraindication to the performance of venography.
10. Any treatment within 2 weeks prior to enrollment in the run-in phase or planned during the run-in phase and/or double-blind treatment phase that could affect the incidence of VTE, such as:
   - parenteral anticoagulants (UFH, LMWH [e.g. enoxaparin, dalteparin, nadroparin, ...], fondaparinux, bivalirudin, hirudin),
   - oral anticoagulants (vitamin K antagonists),
   - GPIIb/IIIa antagonists (abciximab, eptifibatide, tirofiban),
   - thrombolytic agents,
   - dextrans,
   - intermittent pneumatic compression,
10A1. Patient with significant loss of mobility prior to fracture or with any severe medical conditions requiring a prolonged venous thromboprophylaxis according to the investigator's judgment.
11. Known progressive malignant disease.
12. Subject unlikely to comply with protocol (e.g. uncooperative attitude, inability to return for follow-up visits, inability to receive daily injection by a Health Care Professional) after hospital discharge and unlikelihood of completing the study.
13. Treatment with any Investigational Product or investigational device in the last 30 days or 5 half lives (whichever is longer, if relevant) prior to enrollment/randomization.
14. Any previous exposure to AVE5026 (e.g. participation in any previous AVE5026 clinical trial).
15. Refusal or inability to give informed consent to participate in the study.
16. History of heparin-induced thrombocytopenia.
17. Know hypersensitivity to UFH or LMWH.
18. End stage renal disease (estimated creatinine clearance <10 mL/min) or patient on dialysis.
19. Pregnant or breast-feeding women.
20. Women of childbearing potential not protected by effective contraceptive method of birth control as defined for contraception in the Informed Consent Form for the duration of the study and/or who are unwilling or unable to be tested for pregnancy.

Exclusion criteria related to the run-in phase (checked before randomization):
21. Occurrence of any of the exclusion criteria above.
22. Withdrawal of consent.
23. Permanent discontinuation of AVE5026 during the run-in phase.

### 4) Treatments

Sanofi-aventis supplies and manufactures the blinded treatments for this study. According to their randomized assignments, patients receive s.c. either AVE5026 or placebo, once daily. Both treatments are presented as a ready-to-use 0.5 ml prefilled syringe, identical in appearance, and containing the same volume of a sterile, isotonic solution with sodium chloride 0.9 % and water for injection. The dose is 20 mg once-daily for AVE5026, the dose being lowered to 10 mg in case of patients with SRI (estimated CLcr less than 30 mL/min).

Run-in phase (Period I): AVE5026 is administered in an open-label manner once daily for 7 to 10 days after surgery (Day 1 of Period I is the day of surgery). The first post-operative injection of AVE5026 is administered 8 ± 1 hours after incision closure, provided that hemostasis has been established. The second injection of AVE5026 is performed at least 12 hours after the first one, but no later than 24 ± 2 hours. Then, all subsequent injections of AVE5026 are administered 24 ± 2 hours from the previous one.

Double-blind treatment phase (Period II): Investigational Product (IP) of the double-blind treatment phase is administered in a blinded manner once daily for 19 to 23 additional days. Patients receive either AVE5026 or placebo. The first IP injection (AVE5026 or placebo) is administered no later than 24 ± 2 hours from the last injection of the run-in phase. If the timing of the injection has to be reset for practical reasons, the first IP injection should be administered at least 12 hours after the last injection of the run-in phase. Then, all subsequent injections of AVE5026 are administered 24 ± 2 hours from the previous one.

### 5) Results

The efficacy analysis period is defined as the period from randomization up to Day 24 of the double-blind period or up to the mandatory bilateral venography, whichever comes first.

The primary efficacy population includes all randomized patients who received at least one IP injection (active or placebo) and with a non-missing primary efficacy endpoint.

The primary analysis compares the two groups (semuloparin and placebo) using an exact 2-sided stratified test at a α level of 0.05 (Gart 1970). Event rates per treatment group are summarized.

Tables 1 and 2 describe the primary efficacy analysis of the SAVE-HIP3 study.

**Table 1: Any VTE or death during the efficacy analysis period (day of randomization up to end of double-blind treatment phase, i.e. Day 7-10 up to Day 26-33, starting from the day of surgery)**

| | Semuloparin | Placebo |
|---|---|---|
| | (N = 230) | (N = 102) |
| Any VTE or death: | | |
| n (%) | 9 (3.9%) | 19 (18.6%) |
| Comparison versus enoxaparin: | | |
| . Common OR (95% exact CI) | | 0.18 (0.07 to 0.45) |
| . p-value | | <0.0001 |

| | | |
|---|---|---|
| N: number of patients in the primary efficacy population n: number of patients showing a given event | | |

**Table 2: Components of the primary efficacy endpoint**

| | Semuloparin | Placebo | OR |
|---|---|---|---|
| | | | (95% mid-p Cl) |
| Any DVT | | | |
| n/N (%) | 9/230 (3.9%) | 17/100 (17.0%) | 0.20 (0.08 to 0.46) |
| Any proximal DVT | | | |
| n/N (%) | 4/267 (1.5%) | 11/127 (8.7%) | 0.16 (0.04 to 0.50) |
| Distal DVT only | | | |
| n/N (%) | 4/231 (1.7%) | 6/102 (5.9%) | 0.28 (0.07 to 1.06) |
| Non-fatal PE | | | |
| n/N (%) | 0/312 (0%) | 0/157 (0%) | NA |
| All cause death | | | |
| n/N (%) | 0/312 (0%) | 2/157 (1.3%) | 0.00 (0.00 to 1.74) |

| | | | |
|---|---|---|---|
| N: number of efficacy evaluable patients n: number of patients showing a given event NA: non applicable | | | |

These results demonstrate that a 3-week extended prophylaxis with semuloparin, after an initial treatment period of 7-10 days with semuloparin, is more effective than placebo in preventing VTE (DVT, non-fatal PE and death), in particular any DVT and any proximal DVT, in patients having undergone hip fracture surgery.

## Claims

1. Semuloparin or a pharmaceutically acceptable salt thereof, for use in the extended prevention of a mortality and/or morbidity event in a patient having undergone hip fracture surgery, wherein said event is selected from venous thromboembolism and death and wherein the efficacy of said use is clinically proven by a phase III clinical trial.

2. Semuloparin or a pharmaceutically acceptable salt thereof for the use according to claim 1, for use in the extended prevention of venous thromboembolism, including deep vein thrombosis, non-fatal pulmonary embolism and death.

3. Semuloparin or a pharmaceutically acceptable salt thereof for the use according to claim 1 or claim 2, for use in the extended prevention of deep vein thrombosis.

4. Semuloparin or a pharmaceutically acceptable salt thereof for the use according to claim 3, for use in the extended prevention of proximal deep vein thrombosis.

5. Semuloparin or a pharmaceutically acceptable salt thereof for the use according to claim 3, for use in the extended prevention of distal deep vein thrombosis.

6. Semuloparin according to any of claims 1 to 5, wherein semuloparin is administered at a 20 mg daily dose to patients with normal renal function or to patients with mild or moderate renal impairment.

7. Semuloparin or a pharmaceutically acceptable salt thereof for the use according to any of claims 1 to 5, wherein semuloparin is administered at a 10 mg daily dose in patients with severe renal impairment.

8. Semuloparin or a pharmaceutically acceptable salt thereof for the use according to any of claims 1 to 7, wherein semuloparin is administered once daily.

9. Semuloparin or a pharmaceutically acceptable salt thereof for the use according to any of claims 1 to 8, wherein semuloparin is administered for 26 to 33 days, starting from the day of surgery.
